(19) Europäisches Patentamt
European Patent Office
Office européen des brevets

(11) EP 1 844 816 A2

(12) DEMANDE DE BREVET EUROPEEN

(43) Date de publication:
**17.10.2007 Bulletin 2007/42**

(51) Int Cl.:
***A61Q 5/06*** (2006.01) ***A61K 8/44*** (2006.01)

(21) Numéro de dépôt: **07105524.8**

(22) Date de dépôt: **03.04.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **13.04.2006 FR 0603317**

(71) Demandeur: **L'Oréal**
**75008 Paris (FR)**

(72) Inventeurs:
- **SAMAIN, Henri**
  **91570, BIEVRES (FR)**
- **HERCOUET, Leïla**
  **93360, NEUILLY PLAISANCE (FR)**

(74) Mandataire: **Fevrier, Murielle Françoise E.**
**L'OREAL - D.I.P.I. - River Plaza**
**25-29 Quai Aulagnier**
**92665 Asnières-sur-Seine (FR)**

# (54) Composition de coloration comprenant au moins un monomère electrophile, un colorant hydrophile et un solvant organique liquide

(57) La présente invention a pour objet une composition de coloration des matières kératiniques, en particulier des fibres kératiniques telles que les cheveux comprenant au moins un monomère électrophile, au moins un colorant hydrophile particulier et au moins un solvant organique liquide. L'invention concerne également un procédé de coloration de matière kératiniques ainsi que l'utilisation de ladite composition pour colorer les matières kératiniques.

La composition conforme à la présente invention permet d'améliorer la tenacité de la coloration de matières kératiniques vis-à-vis des agressions extérieures tout en présentant une large gamme de nuances et sans dénaturer les matières kératiniques.

EP 1 844 816 A2

## Description

La présente invention a pour objet une composition de coloration des matières kératiniques, en particulier des fibres kératiniques telles que les cheveux, comprenant au moins un monomère électrophile, au moins un colorant hydrophile particulier et au moins un solvant organique liquide. L'invention concerne également un procédé de coloration de matière kératiniques ainsi que l'utilisation de ladite composition pour colorer les matières kératiniques.

Depuis plusieurs années, des recherches scientifiques sont menées pour modifier la couleur de matières kératiniques notamment des fibres kératiniques et en particulier pour masquer les fibres blanches.

Dans le domaine de la coloration des fibres kératiniques, il est déjà connu de colorer des fibres kératiniques par différentes techniques à partir de colorants directs ou de pigments pour des colorations non permanente ou de précurseurs de colorant pour des colorations permanentes.

La coloration d'oxydation consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées. La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Cependant les agents oxydants utilisés dégradent les propriétés cosmétiques et mécaniques des fibres en cas de colorations répétées ce qui peut entraîner un coiffage ou une mise en forme difficile.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une résistance aux shampooings non satisfaisante.

Par ailleurs, il est connu de teindre les fibres kératiniques et en particulier les cheveux, avec des compositions de teinture contenant des colorants directs. Cette technique est appelée coloration non permanente ou coloration directe.

Ces colorants sont des molécules colorées et colorantes, qui doivent présenter une certaine affinité pour les fibres kératiniques. Ils sont appliqués sur les fibres kératiniques pendant un temps nécessaire à l'obtention de la coloration désirée, puis rincés.

Les colorations qui en résultent sont des colorations souvent chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs il est connu de la demande de brevet FR 2 833 489 des compositions de traitement des cheveux à partir de compositions comprenant des monomères électrophiles capables de se polymériser par voie anionique directement sur les cheveux. Ces monomères après polymérisation permettent d'obtenir des cheveux parfaitement gainés. Cependant le gainage obtenu à partir des monomères électrophiles ne présente pas une résistance satisfaisante par rapport aux différentes agressions que peuvent subir les cheveux telles que les shampooings, le sébum, les frottements, la lumière, les intempéries, la sueur et les déformations permanentes.

Ainsi, il existe toujours un besoin de développer de nouvelles compositions de teinture directe en vue d'obtenir des nuances variées, en particulier dans des nuances pastels, qui présentent une bonne ténacité, notamment aux agents extérieurs tels que la lumière, le shampooing, la sueur et qui permettent de surcroît de conserver l'intégrité des fibres kératiniques.

En particulier, il existe un besoin de développer des compositions de coloration permettant d'obtenir des colorations présentant une ténacité proche de la coloration par oxydation sans les inconvénients liés à la présence d'un agent oxydant.

Ce but est atteint avec la présente invention qui a pour objet une composition de coloration comprenant au moins un monomère électrophile ; au moins un colorant direct hydrophile possédant un LogP, inférieur ou égal à 2 ; et au moins un solvant organique liquide.

Une telle composition tinctoriale permet notamment d'exacerber la rémanence dudit colorant sur la matière kératinique en l'associant à un monomère électrophile polymérisable.

Il se forme ainsi à la surface du cheveu un gainage rémanent aux agressions extérieures, telles que le sébum, la lumière, le shampooing.

Le gainage ainsi obtenu est homogène, lisse et possède une excellente adhésion sur cheveux.

Le polymère formé enrobe ainsi le colorant hydrophile formant un gainage coloré rémanent aux agents extérieurs.

Les cheveux ainsi traités restent parfaitement individualisés et peuvent être coiffés sans problème.

De plus l'invention permet d'obtenir des nuances variant du pastel à des colorations intenses tout en restant résistante aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis-à-vis des shampooings.

La présente invention a aussi pour objet un procédé de coloration des matières kératiniques qui comprend l'application sur les matières kératiniques de la composition de l'invention ainsi que l'utilisation de la composition de l'invention pour la coloration des matières kératiniques, en particulier les fibres kératiniques telles que les cheveux.

Elle a également pour objet un kit comprenant dans un compartiment la composition contenant au moins un monomère électrophile et au moins un colorant direct possédant un LogP inférieur ou égal à 2, et dans une autre compartiment une deuxième composition qui contient au moins un agent nucléophile ; étant entendu que le solvant peut se trouver dans l'un et/ou l'autre des compartiments.

Une variante est que le kit comprenne dans un compartiment la composition contenant au moins un monomère électrophile, et dans un autre compartiment une deuxième composition qui contient au moins un colorant direct possédant un LogP inférieur ou égal à 2 ; étant entendu que le solvant peut se trouver dans l'un et/ou l'autre des compartiments, de préférence avec le monomère électrophile.

Le ou les monomères électrophiles présents dans la composition de l'invention peuvent être choisis parmi :

- les dérivés benzylidène malononitrile (A1), le 2-(4-chlorobenzylidène)-malononitrile (A2) le 2-cyano-3-phényl acrylate d'éthyle (B1) le, 2-cyano-3-(4-chloro-phényl) acrylate d'éthyle (B2) décrits dans Sayyah, J. Polymer Research, 2000, p97 ;

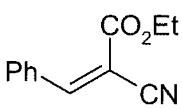
(A1)
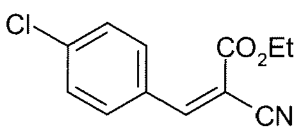
(A2)
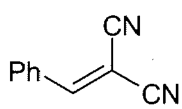
(B1)
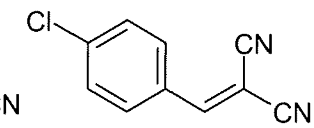
(B2)

- les dérivés de méthylidènemalonates comme :

  ➢ Le 2-méthylène-malonate de diéthyle (C1) par Hopff, Makromoleculare Chemie, 1961, p95, De Keyser, J. Pharm. Sci, 1991, p67 et Klemarczyk, Polymer, 1998, p173

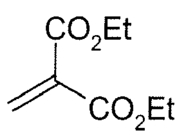
(C1)

  ➢ le 2-éthoxycarbonylméthylèneoxycarbonyl acrylate d'éthyle (D1) par Breton, Biomaterials, 1998, p271 et Couvreur, Pharmaceutical Research, 1994, p1270.

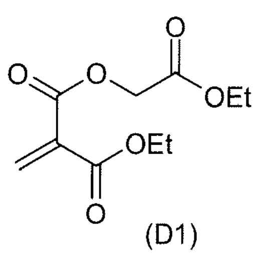
(D1)

- Les dérivés itaconate et itaconimide comme :

  ➢ l'itaconate de diméthyle (E1) par Bachrach, European Polymer Journal, 1976, p563

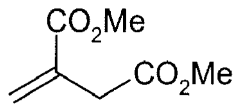

(E1)

  ➢ N-butyl itaconimide (F), N-(4-tolyl) itaconimide (G), N-(2-éthylphényl) itaconimide (H) , N-(2,6-diéthylphényl) itaconimide (I) par Wanatabe, J.Polymer Science : Part A :Polymer chemistry, 1994, p2073

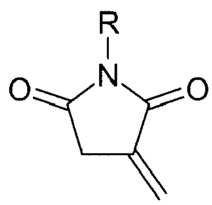

R= Bu (F),

4-tolyl (G), 2-éthylphényl (H), 2,6-diéthyphényl (I)

- Les dérivés α-(méthylsulfonyl)acrylates de méthyle (K), α-(méthylsulfonyl)acrylates de d'éthyle (L), α-(tert-butylsulfonyl)acrylates de méthyle (M), α-(méthylsulfonyl)acrylates de tert-butyle (N), α-(tert-butylsulfonyl)acrylates de tert-butyle (O) par Gipstein, J.Org.Chem, 1980, p1486 et les dérivés 1,1-bis-(méthylsulfonyl)éthylène (P), 1-acétyl-1-méthylsulfonyléthyléne (Q), α-(méthylsulfonyl)vinyl sulfonate de methyle (R), α-methylsulfonylacrylonitrile (S) par Shearer, US patent US2748050.

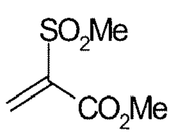

(K)

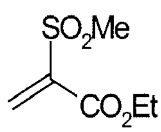

(L)

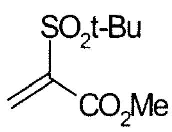

(M)

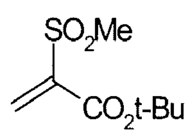

(N)

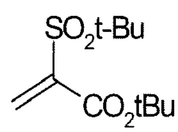

(O)

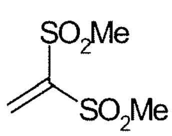

(P)

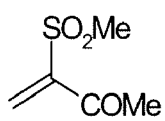

(Q)

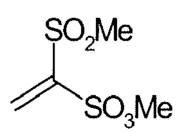

(R)

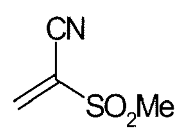

(S)

- Les dérivés méthyl vinyl sulfone (T) et phénylvinyl sulfone (U) par Boor , J.Polymer Science, 1971, p249

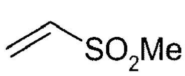

(T)

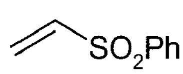

(U)

- Le dérivé phényl vinyl sulfoxide (V) par Kanga, Polymer preprints (ACS, Divison of Polymer Chemistry), 1987, p322

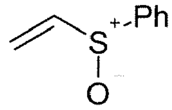

(V)

- Le dérivé 3-méthyl-N-(phenylsulfonyl)-1-aza-1,3-butadiène (W) par Bonner, Polymer Bulletin, 1992, p517

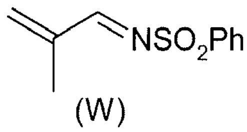

(W)

- Les dérivés acrylates et acrylamides comme :

  ➢ N-propyl-N-(3-triisopropoxysilylpropyl)acrylamide (X) et N-propyl-N-(3-triethoxysilylpropyl)acrylamide (Y) par Kobayashi, Journal of Polymer Science, Part A: Polymer Chemistry, 2005, p2754.

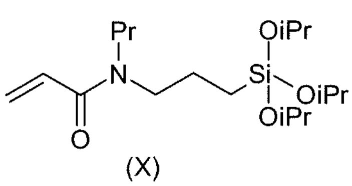

(X)

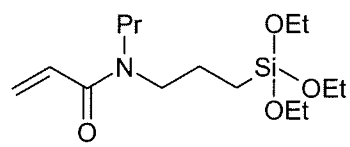

(Y)

  ➢ 2-hydroxyethyl acrylate (Z) et 2-hydroxyethyl méthacrylate (AA) par Rozenberg, International Journal of Plastics Technology, 2003, p17

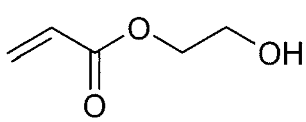

(Z)

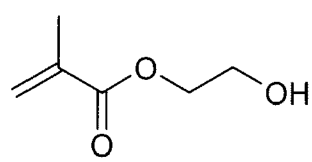

(AA)

  ➢ N-butyl acrylate (AB) par Schmitt, Macromolecules, 2001, p2115
  ➢ Tert-butyl acrylate (AC) par Ishizone, Macromolecules, 1999, p955.

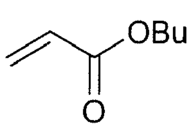

(AB)

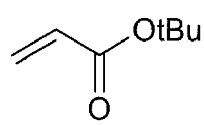

(AC)

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (A) :

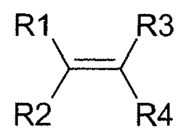

(A)

dans laquelle :

- R1 et R2 désignent chacun, indépendamment l'un de l'autre, un groupe peu ou non électro-attracteur (peu ou non inductif-attracteur) tel que :
  - un atome d'hydrogène,
  - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SR, et les atomes d'halogène,
  - un résidu polyorganosiloxane modifié ou non,
  - un groupement polyoxyalkylène, de préférence R1 et R2 représentent un atome d'hydrogène ;

- R3 et R4 désignent chacun, indépendamment l'un de l'autre, un groupe électro-attracteur (ou inductif-attacteur) choisi de préférence parmi les groupements $-N(R)_3^+$, $-S(R)_2^+$-, $-SH_2^+$, $-NH_3^+$, $-NO_2$, $-SO_2R$, -C≡N, , -COOH, -COOR, -COSR, $-CO(NH_2)$, -CONHR, $-CON(R)_2$, -F, -Cl, -Br, -I, -OR, -COR, SH, -SR, -OH, les groupes alcényle linéaires ou ramifiés, les groupes alcynyle linéaires ou ramifiés, les groupements mono- ou polyfluoroalkyle en $C_1$-$C_4$, les groupements aryle tels que phényle, ou les groupements aryloxy tels que phénoxyloxy,
- R, désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que $-(CH_2)_n-(CF_2)_m-CF_3$ ou $-(CH_2)_n-(CF_2)_m-CHF_2$ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Parmi les monomères précédemment cités, sont préférés les monomères de la famille des cyanoacrylates et leurs dérivés de formule (B) :

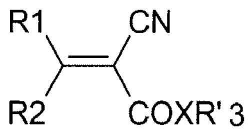
(B)

X désignant NH, S ou O,
R1 et R2 ayant les mêmes significations que précédemment, de préférence R1 et R2 représentant un atome d'hydrogène,
R'3 représentant un atome d'hydrogène ou R tel que défini à la formule (A),

De préférence, X désigne O.

De préférence R'3 représente un radical alkyle, linéaire ou ramifié, comprenant de 6 à 10 atomes de carbone ou alcényle comprenant de 2 à 10 atomes de carbone.

A titre de composés de formule (B), on peut citer les monomères :

a) appartenant à la famille des 2-cyanoacrylates de polyfluoroalkyle tels que :

l'ester 2,2,3,3-tétrafluoropropylique de l'acide 2-cyano-2-propénoïque de formule (D) :

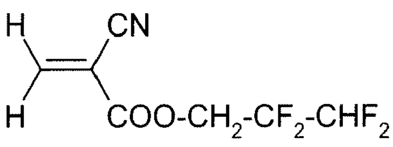
(D)

ou encore l'ester 2,2,2-trifluoroéthylique de l'acide 2-cyano-2-propénoïque de formule (E) :

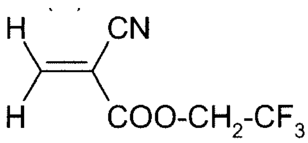
(E)

b) les 2-cyanoacrylates d'alkyle ou d'alcoxyalkyle de formule (C) :

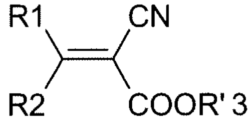
(C)

dans laquelle
R1 et R2 ayant les mêmes significations que précédemment, de préférence R1 et R2 représentant un atome

d'hydrogène,
R'3 représente un radical alkyle en $C_1$-$C_{10}$ ou alcoxy($C_1$-$C_4$) alkyle($C_1$-$C_{10}$) et alcényle en $C_2$-$C_{10}$.

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le cyanoacrylate d'iso-amyle, 2-cyanoacrylate d'allyle et le 2-cyanoacrylate de méthoxypropyle.

Dans le cadre de l'invention, on préfère utiliser les monomères b) de formule (C). Selon un mode de réalisation préféré, le ou les monomères cyanoacrylates sont choisis parmi les cyanoacrylates d'alkyle en $C_6$-$C_{10}$ ou alcényle en $C_2$-$C_{10}$.

Les monomères particulièrement préférés sont les cyanoacrylates d'octyle de formule (F) et leurs mélanges :

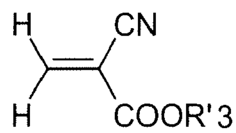
(F)

dans laquelle : $R'_3$ =$-(CH_2)_7-CH_3$,
$-CH(CH_3)-(CH_2)_5-CH_3$,
$-CH_2-CH(C_2H_5)-(CH_2)_3-CH_3$,
$-(CH_2)_5-CH(CH_3)-CH_3$,
$-(CH_2)_4-CH(C_2H_5)-CH_3$.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les monomères électrophiles peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Dans la composition de l'invention, le monomère électrophile conforme à l'invention est introduit en une quantité comprise de préférence entre 0,1 et 99.9 % en poids du poids total de la composition, de préférence entre 1 et 50 %.

Dans le cadre de l'invention, les monomères électrophiles sont des monomères capables de polymériser par voie anionique en présence d'un agent nucléophile.

Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par "carbanion", les espèces chimiques définies dans "Advanced Organic Chemistry," Third Edition", de Jerry March, page 141.

Les agents nucléophiles peuvent être appliqués indépendamment de la composition de l'invention. Ils peuvent aussi être ajoutés à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : $R_2N^-$, $NH_2^-$, $Ph_3C^-$, $R_3C^-$, $PHNH^-$, pyridine, $ArS^-$, $R-C\equiv C^-$, $RS^-$, $SH^-$, $RO^-$, $R_2NH$, $ArO^-$, $N_3^-$, $OH^-$, $ArNH_2$, $NH_3$, $I^-$, $Br^-$, $Cl^-$, $RCOO^-$, $SCN^-$, ROH, RSH, $NCO^-$, $CN^-$, $NO_3^-$, $ClO_4^-$, $H_2O$, Ph représentant un groupe phényle ; Ar représentant un groupe aryle et R représentant un groupe alkyle en $C_1$-$C_{10}$.

De préférence l'agent nucléophile est l'eau.

Dans le cadre de l'invention, la valeur du logP représente de façon classique le coefficient de partage du colorant entre l'octanol et l'eau. La valeur du logP peut être calculée selon la méthode décrite dans l'article de Meylan et Howard « Atom / Fragment contribution method for estimating octanol-water partition coefficient », J. Pharm. Sci., 1995, 84, p83-92. Cette valeur peut aussi être calculée à partir de nombreux logiciels disponibles sur le marché qui détermine la valeur de logP en fonction de la structure d'une molécule.

A titre d'exemple, on peut citer le logiciel Epiwin de l'agence de l'environnement des Etats-Unis.

Les colorants directs qui peuvent être utilisés dans la composition de l'invention sont des colorants hydrophiles

connus présentant une valeur LogP inférieure ou égale à 2.

De façon avantageuse, LogP est strictement inférieur à 2.

Les colorants directs utilisables selon l'invention sont choisis de préférence parmi les colorants directs nitrés benzéniques neutres ; acides ou cationiques ; les colorants directs azoïques neutres acides ou cationiques ; les colorants directs quinoniques et en particulier anthraquinoniques neutres, acides ou cationiques ; les colorants directs aziniques ; les colorants directs triarylméthaniques ; les colorants directs indoaminiques et les colorants directs naturels.

Les colorants directs peuvent porter deux ou plus de deux fonctions cationiques ou anioniques.

Le colorant hydrophile est notamment choisi de telle manière à être solubilisé ou dispersé dans la composition.

Ainsi, les colorants directs peuvent être choisis en fonction de la valeur LogP.
A titre d'exemples, on peut notamment citer les colorants suivants :

| STRUCTURE | NOM | log P |
|---|---|---|
| 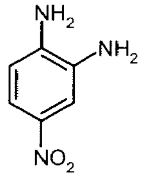 | 4-nitro-o-phénylène-diamine | 0,88 |
| 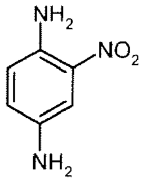 | 2-nitro-p-phénylène-diamine | 0,53 |
| 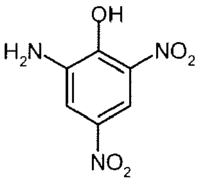 | acide picramique | 0,93 |
| 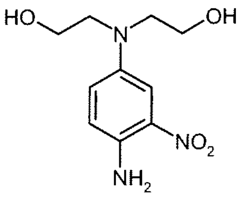 | HC Red 13 | 0,66 |
| 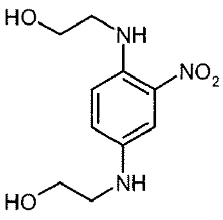 | N,N'-bis-(2-hydroxyéthyl)-2-nitro-p-phénylène-diamine | -0,44 |
| 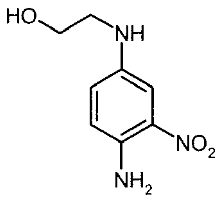 | HC Red 7 | 0,13 |

(suite)

| STRUCTURE | NOM | log P |
|---|---|---|
| 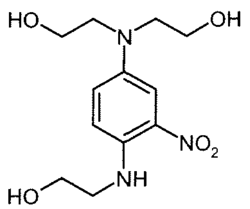 | HC Blue 2 | -0,32 |
| 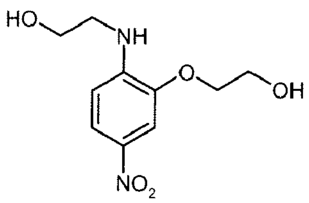 | HC Yellow 4 | 0,56 |
| 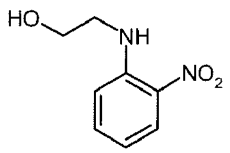 | HC Yellow 2 | 1,05 |
| 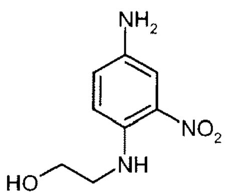 | HC Red 3 | -0,42 |
| 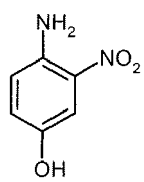 | 4-amino-3-nitrophénol | 1,19 |
| 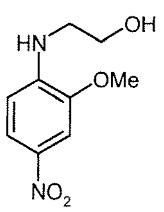 | 1-hydroxyéthyl-amino-5-nitroanisole | 1,13 |
| 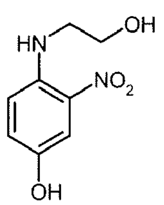 | 3-nitro-p-hydroxyéthylamino phénol | 0,21 |
| 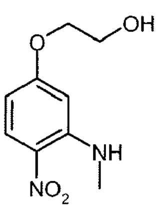 | 3-méthylamino-4-nitrophénoxyéthanol | 1,13 |

(suite)

| STRUCTURE | NOM | log P |
|---|---|---|
| 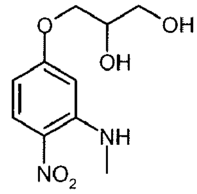 | 2-nitro-5-glycéryl méthylaniline | 0,89 |
| 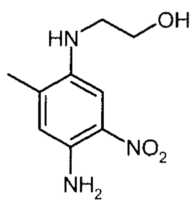 | HC Violet 1 | 0,67 |
| 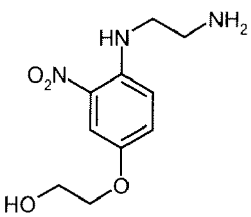 | HC Orange 2 | 0,15 |
| 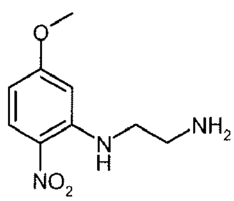 | HC Yellow 9 | 1,12 |
| 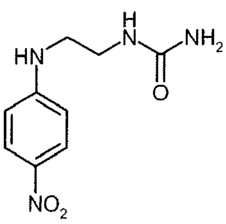 | 4-nitrophényl aminoéthylurée | 0,59 |
| 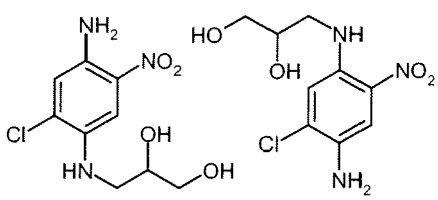 | HC Red 10 et HC Red 11 | 0,13 |
| 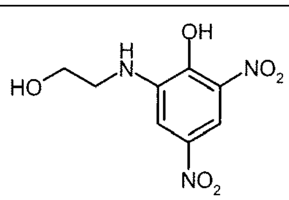 | Acide 2-hydroxyéthyl picramique | 0,38 |

(suite)

| STRUCTURE | NOM | log P |
|---|---|---|
| 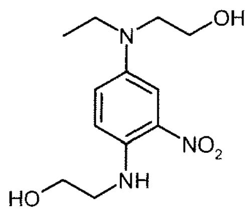 | HC Blue 12 | 1,15 |
| 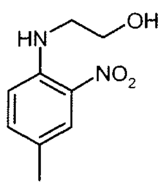 | 3 nitro 4 N beta hydroxyéthyl aminotoluène | 1,59 |
| 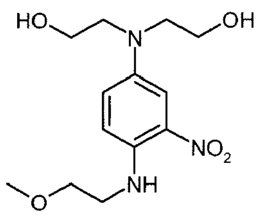 | 2 N béta méthoxyéthylamino 5 N,Nbis (hydroxyéthyl) amino nitrobenzène | 0,38 |
| 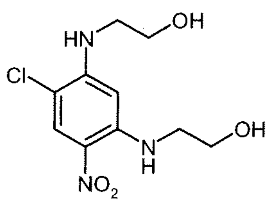 | HC Yellow 10 | 0,20 |
| 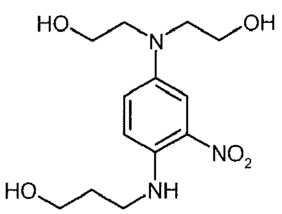 | HC Violet 2 | 0,17 |
| 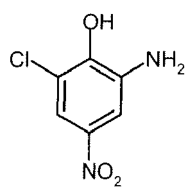 | 2-amino-6-chloro-4-nitrophénol | 1,53 |
| 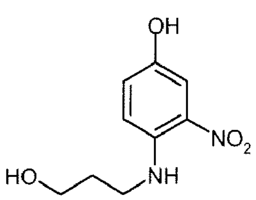 | 4-hydroxypropyl-amino-3-nitrophénol | 0,70 |
| 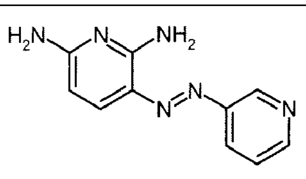 | 2,6-diamino-3-((pyridine-3-yl)-azo)pyridine | 1,58 |

(suite)

| STRUCTURE | NOM | log P |
|---|---|---|
| 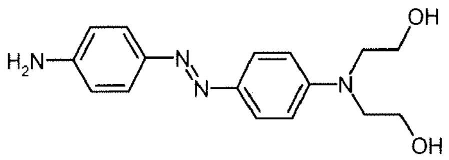 | Disperse Black 9 | 1,83 |
| 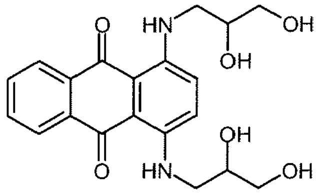 | HC Blue 14 | 0,62 |
| 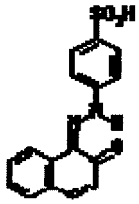 | Acid orange 7 | 1,14 |
| 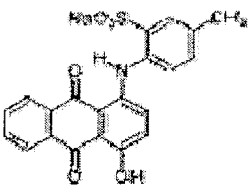 | Acid violet 43 | 1 |
| 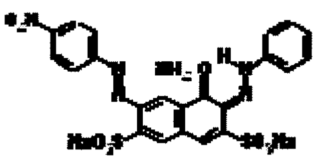 | Acid black 1 | -4,53 |
| 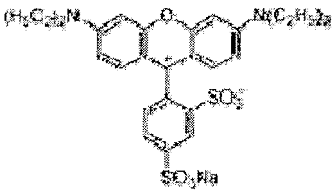 | Acid red 52 | 1,3 |
| 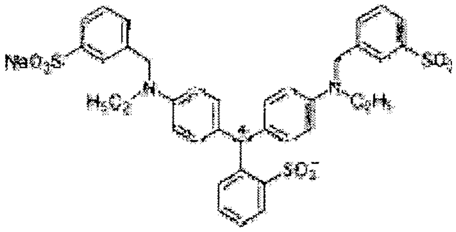 | Acid blue 9 | -0,32 |
| 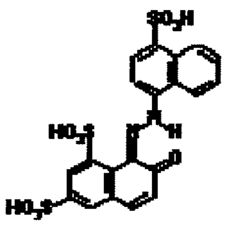 | Acid red 18 | 1,63 |

(suite)

| STRUCTURE | NOM | log P |
|---|---|---|
| 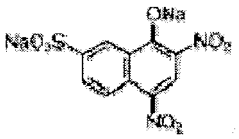 | Acid yellow 1 | 1,22 |

Le ou les colorants directs présentant une valeur de logP inférieure ou égale à 2 peuvent être présents dans la composition dans des quantités comprises entre 0,001 à 10 % en poids environ du poids total de la composition, de préférence entre 0.01 et 10 % en poids total de la composition.
Le milieu des compositions de l'invention peut contenir au moins un solvant organique liquide.
Par solvant organique, on entend une substance organique capable de dissoudre une autre substance sans la modifier chimiquement.
Les solvants organiques sont choisis parmi les composés liquides à la température de 25°C et sous 105 Pa (760 mm de Hg) et sont différents des monomères électrophiles selon l'invention.
Le solvant organique est par exemple choisi parmi :

les alcools aromatiques tels que l'alcool benzylique ; les alcools gras liquides , notamment en $C_{10}$-$C_{30}$; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de $C_5$ à $C_{10;}$ les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en $C_5$-$C_{10}$, l'acétone, la méthyléthylcétone, les esters d'acides en $C_1$-$C_{20}$ liquides et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras liquides en $C_{10}$-$C_{30}$ tels que l'alcool oléique, les esters d'alcools gras en $C_{10}$-$C_{30}$ liquides tels que les benzoates d'alcool gras en $C_{10}$-$C_{30}$ et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions $\alpha$ et $\omega$ (85,3% en poids), ou leurs mélanges.
Selon un mode de réalisation préféré, le solvant organique est constitué par une silicone ou un mélange de silicones tels que les polydiméthylsiloxanes liquides et les polydiméthylsiloxanes modifiées liquides, la viscosité du silicone ou du mélange de silicones à 25 °C est comprise entre 0.1 cst et 1 000 000 cst et plus préférentiellement entre 1 cst et 30 000 cst.
On citera de préférence les huiles et mélanges d'huiles suivantes :

- le mélange de polydiméthylsiloxane alpha-omega-dihydroxylé/cyclopentadiméthylsiloxane (14,7/85,3) commercialisé par Dow Corning sous le nom de DC 1501 Fluid ;
- le mélange de polydiméthylsiloxane alpha-omega-dihydroxylé/ polydiméthylsiloxane commercialisé par Dow Corning sous le nom de DC 1503 Fluid ;
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;

et les mélanges respectifs de ces huiles.
Le milieu des compositions de l'invention peut contenir, outre le ou les solvants organiques liquides, de l'eau. De préférence c'est un milieu anhydre c'est-à-dire contenant moins de 1 % en poids d'eau par rapport au poids total de la composition.
Le ou les solvants organiques de la composition représentent généralement de 0,01 à 99 %, de préférence de 50 à 99 % en poids par rapport au poids total de la composition.

La composition de l'invention peut aussi se présenter sous forme d'une émulsion et/ou être encapsulé, les monomères électrophiles étant maintenus dans un milieu anhydre jusqu'au moment de l'utilisation. Lorsque la composition de l'invention est une émulsion, cette émulsion est par exemple constituée par une phase dispersée ou continue qui peut être constituée par de l'eau, des alcools aliphatiques en $C_1$-$C_4$ ou leurs mélanges et une phase organique anhydre comprenant le monomère. Dans le cas des capsules ou microcapsules, la capsule peut contenir le monomères dans un milieu anhydre et être dispersées dans un milieu anhydre tel que défini précédemment, de l'eau, des alcools aliphatiques en $C_1$-$C_4$ ou leurs mélanges.

On peut également introduire dans la composition de l'invention des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que l'hydroquinone monéthyléther, la tertiobutylhydroquinone, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, et leurs mélanges. Les groupements alkyles désignent de préférence des groupements ayant 1 à 6 atomes de carbone.

La concentration en inhibiteur dans la composition de l'invention peut être comprise entre 10 ppm et 30 %, et plus préférentiellement entre 10 ppm et 15% en poids.

On peut aussi utiliser à titre d'inhibiteur les acides minéraux ou organiques.

Les acides sont notamment des acides minéraux ou organiques, ces derniers ayant un ou plusieurs groupements carboxyliques ou sulfoniques, et présentant un pKa compris entre 0 et 6.

A titre d'exemple, on peut citer l'acide phosphorique, l'acide chlorhydrique, l'acide nitrique, un acide sulfonique tel que l'acide benzène- ou toluène-sulfonique, l'acide sulfurique, l'acide carbonique, l'acide fluorhydrique, l'acide acétique, l'acide formique, l'acide propionique, l'acide octanoïque, l'acide heptanoïque, l'acide hexanoique, l'acide benzoïque, les acides mono-, di- ou trichloroacétiques, l'acide salicylique et l'acide trifluoroacétique.

De préférence, l'acide est un acide organique.

A titre d'acide organique préféré, on peut citer l'acide acétique.

Lorsqu'il est présent, l'acide est généralement présent en quantité comprise de préférence entre 0,01% et 30% en poids de la composition, de préférence entre 0,01 % et 15% en poids de la composition.

La composition de l'invention peut aussi contenir un ou plusieurs pigments classiques. Elle peut aussi contenir des poudres ou particules métalliques telles que des poudres ou particules d'aluminium, de zinc, de cuivre, etc.

La composition peut aussi contenir les actifs cosmétiques habituellement utilisés. On peut citer à titre non limitatif les charges, les polymères, les élastomères, les polymères fixants ou non, les polymères conditionneurs, les pigments organiques ou minéraux, les nacres, les colorants directs autres que les colorants hydrophiles cités dans l'invention, les colorants d'oxydation, les agents réducteurs, les agents oxydants, les corps gras, les silicones, les agents épaississants, les adoucissants, les agents anti-mousse, les agents hydratants, les agents émollients, les agents alcalinisants, les plastifiants, les filtres solaires, les argiles, les minéraux colloïdaux, les parfums, les peptisants, les conservateurs, les tensioactifs anioniques cationiques, amphotères, zwitterioniques ou non-ioniques, les protéines, les vitamines...

Afin d'améliorer entre autre l'adhésion du polymère formé in situ par réaction des monomères électrophiles selon l'invention, la fibre peut être prétraitée avec tous types de polymères.

Ces compositions peuvent se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Selon le procédé de l'invention, un mode de réalisation préféré consiste à appliquer sur la matière kératinique, en particulier sur la fibre kératinique telle que le cheveu, la composition selon l'invention contenant le monomère électrophile et les colorants hydrophiles comprenant un logP inférieur ou égal à 2.

Plus spécifiquement la composition de l'invention est appliquée sur les matières kératiniques, en particulier les fibres kératiniques telles que les cheveux, en présence d'un agent nucléophile.

Selon un mode de réalisation particulier du procédé de l'invention, l'agent nucléophile capable d'initier la polymérisation du monomère cyanoacrylate peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de l'invention peut être mise en oeuvre en plusieurs étapes : une première étape qui consiste à appliquer une composition contenant le ou les colorants hydrophiles comprenant un logP inférieur ou égal à 2 sur les fibres et une seconde étape qui consiste à appliquer une composition contenant le monomère électrophile, l'agent nucléophile étant présent dans une composition séparée. Un rinçage intermédiaire ou d'autres traitements peuvent être appliqués.

On peut également espacer dans le temps les deux étapes.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) formé *in situ,* la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfures composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

L'application de la composition de l'invention peut aussi être précédée d'un traitement capillaire comme une coloration directe ou d'oxydation.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C ce qui n'empêche pas de terminer l'application par un séchage au casque, un brushing ou passage au fer plat ou à friser.

L'invention a aussi pour objet un kit de coloration comprenant dans un compartiment une première composition qui contient le ou les monomères électrophiles et au moins un colorant hydrophile direct et dans un autre compartiment une deuxième composition qui contient au moins un agent nucléophile, étant entendu que l'un et/ou l'autre des compartiment contient au moins un solvant organique liquide, de préférence avec le monomère électrophile. Selon ce mode de réalisation, la composition contenant le ou les colorants hydrophiles directs et le ou les monomères électrophiles, peut être une composition anhydre.

L'invention a aussi pour objet un kit de coloration comprenant dans un compartiment une première composition qui contient le ou les monomères électrophiles et dans un autre compartiment une deuxième composition qui contient au moins un colorant hydrophile direct, l'un et/ou l'autre contenant au moins un solvant organique liquide, de préférence avec le monomère électrophile. Selon ce mode de réalisation, la composition contenant le ou les monomères électrophiles, peut être une composition anhydre.

Les exemples suivants non limitatifs permettent d'illustrer l'invention sans en limiter sa portée.

## EXEMPLES

Des essais ont été réalisés en utilisant les compositions suivantes :

### 1- HC Red 13

La composition suivante est réalisée :

| | |
|---|---|
| DC 1501 Fluid | 40g |
| DC 245 Fluid | 39g |
| HC Red 13 (logP = 0,66) | 1g |
| Méthylheptylcyanoacrylate de Chemence | 10g |

(suite)

| | |
|---|---|
| Acide acétique* | 0.25g |

0,5g de la composition est appliqué sur une mèche de 1g de cheveux propre et humide. Après 15 minutes de pause, la mèche est séchée au sèche-cheveux pendant 2 minutes.
La mèche obtenue est colorée en rouge et est résistante aux shampooings.

**2- HC Blue 14**

La composition suivante est réalisée :

| | |
|---|---|
| DC 1501 Fluid | 40g |
| DC 245 Fluid | 39g |
| HC Blue 14 (logP = 0,62) | 1g |
| Méthylheptylcyanoacrylate de Chemence | 10g |
| Acide acétique* | 0.25g |

0,5g de la composition est appliqué sur une mèche de 1 g de cheveux propre et humide. Après 15 minutes de pause, la mèche est séchée au sèche-cheveux pendant 2 minutes.
La mèche obtenue est colorée en bleu et est résistante aux shampooings.

**3- Disperse Black 9**

La composition suivante est réalisée :

| | |
|---|---|
| DC 1501 Fluid | 40g |
| DC 245 Fluid | 39.8g |
| Disperse Black 9 (logP = 1,83) | 0.2g |
| Méthylheptylcyanoacrylate de Chemence | 10g |
| Acide acétique* | 0.25g |

0,5g de la composition est appliqué sur une mèche de 1 g de cheveux propre et humide. Après 15 minutes de pause, la mèche est séchée au sèche-cheveux pendant 2 minutes.
La mèche obtenue est colorée en noir et est résistante aux shampooings.

**4- HC Yellow 10**

La composition suivante est réalisée :

| | |
|---|---|
| DC 1501 Fluid | 40g |
| DC 245 Fluid | 39.5g |
| HC Yellow 10 (logP = 0,20) | 0.5g |
| Méthylheptylcyanoacrylate de Chemence | 10g |
| Acide acétique* | 0.25g |

0,5g de la composition est appliqué sur une mèche de 1g de cheveux propre et humide. Après 15 minutes de pause, la mèche est séchée au sèche-cheveux pendant 2 minutes.
La mèche obtenue est colorée en jaune et est résistante aux shampooings.

**5- Mélange de colorants acides**

La formule colorante « A » est préparée.

Composition de la formule colorante « A »

| | C (g %) |
|---|---|
| Alcool dénaturé | 22,34 |
| Alcool benzylique | 10 |
| Glycérine | 5 |
| Acide citrique | 4 |
| Acide lactique | 1,98 |
| Hydroxyéthyl cellulose | 1,20 |
| Gomme de xanthane | 0,40 |
| PCA sodium | 0,25 |
| Hydroxypropyltrimonium hydrolized wheat protein | 0,03 |
| Phenoxyethanol | 0,0002 |
| Acid orange 7 (LogP=1,14) | 0,1962 |
| Acid violet 43 (LogP=1) | 0,0556 |
| Acid black 1 (LogP=-4,53) | 0,0243 |
| Acid red 52 (LogP=1,3) | 0,0297 |
| Acid blue 9 (LogP=0,01) | 0.01 |
| Acid red 18 (LogP=1,63) | 0,0087 |
| Acid yellow 1 (LogP=1,22) | 0,0056 |
| Eau | Qsp 100 |

Au moment de l'emploi, on mélange dans un récipient en plastique 10 g de la formule « A » et 1 g d'octylcyanoacrylate conduisant au mélange « B ».

Le mélange obtenu « B » est ensuite appliqué sur 1 g de mèche de cheveu à 90 % blanc naturel.

Le temps de pose est de 30 min à température ambiante. A L'issue des 30 min, la mèche est rincée.

La mèche subit ensuite une série de 6, puis 12 shampooings ; pour chaque cycle de shampooing, le protocole suivant est adopté:

1. application sur mèche de 0,4 g de shampooing ELSEVE MULTIVITAMINE et malaxage pendant 10 secondes ;
2. pose de 5 min puis rinçage à l'eau pendant 10 secondes ;
3. séchage au casque.

La couleur des mèches avant et après les lavages a été évaluée dans le système L*a*b* au moyen d'un spectrophotomètre, colorimètre CM 2002 MINOLTA ®, Illuminant D65-10° CSI.

Dans ce système L* a* b*, L* représente l'intensité de la couleur, a* indique l'axe de couleur vert/rouge et b* l'axe de couleur bleu/jaune.

Plus la valeur de L est faible, plus la couleur est foncée ou très intense. Plus la valeur de a* est élevée plus la nuance est rouge et plus la valeur de b* est élevée plus la nuance est bleue.

La perte en couleur $\Delta E$ après shampooing est déterminée selon l'équation suivante* :

$$\Delta E = \sqrt{(L_1 - L_0)^2 + (a_1^* - a_0^*)^2 + (b_1^* - b_0^*)^2}$$

Dans cette équation, $L_1$, $a_1$* et $b_1$* représentent les valeurs colorimétriques des mèches mesurées après shampooings et $L_0$, $a_0$* et $b_0$* représentent les valeurs mesurées avant shampooing.

Plus la valeur de $\Delta E$ est importante, plus la différence de couleur avant et après lavages est importante, et moins la coloration est résistante aux shampooings.

Parallèlement, un témoin est réalisé : on applique 10g de formule colorante « A » sur 1 g de mèche de cheveu à 90% blancs naturel. Les conditions d'application et de mesure sont les mêmes que celles décrites précédemment avec le mélange « B ».

**Résultats obtenus**

**[0131]** - Le mélange « B » n'adhère ni au récipient en plastique, ni aux gants, contrairement à la formule « A ».
- Comme le montre le tableau ci-dessous, la mèche colorée avec le mélange « B » est plus résistante au shampooing que la mèche traitée uniquement avec la formule « A ».

| | Perte ($\Delta E$) après 6 shampooings | Perte ($\Delta E$) après 12 shampooings |
|---|---|---|
| Formule « A » | 7,59 | 12,60 |
| Mélange « B » | 3,48 | 6,19 |

**Revendications**

1. Composition de coloration comprenant au moins un monomère électrophile ; au moins un colorant direct hydrophile possédant un LogP inférieur ou égal à 2 et au moins un solvant organique liquide.

2. Composition selon la revendication 1 dans laquelle le ou les monomères électrophile sont des monomères de formule (A) :

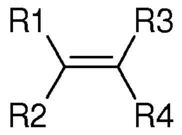

(A)

dans laquelle :

• R1 et R2 désignent chacun, indépendamment l'un de l'autre, un groupe peu ou non électro-attracteur, et
• R3 et R4 désignent chacun, indépendamment l'un de l'autre, un groupe électro-attracteur.

3. Composition selon la revendication 2 dans laquelle les monomères de formule (A) sont tels que R1 et R2, indépendamment l'un de l'autre, représentent un atome d'hydrogène ; un groupe hydrocarboné saturé ou non, linéaire, ramifié ou cyclique, comportant de préférence de 1 à 20, et contenant éventuellement un ou plusieurs atomes d'azote, d'oxygène, de soufre, et éventuellement substitué par un ou plusieurs groupements choisis parmi -OR, -COOR, -COR, -SR et les atomes d'halogène ; un résidu polyorganosiloxane modifié ou non ; un groupement polyoxyalkylène, R désignant un atome d'hydrogène ou un groupe hydrocarboné saturé ou non, linéaire, ramifié ou cyclique, comportant de 1 à 20, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R'désignant un groupe alkyle en $C_1$-$C_{10}$.

4. Composition selon la revendication précédente dans laquelle les monomères sont des monomères cyanoacrylates de formule (B):

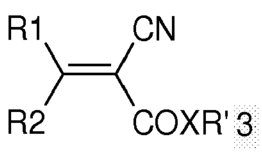

(B)

X désignant NH, S ou O,

$R'_3$ étant choisi parmi un atome d'hydrogène ou R,
R, R1 et R2 étant tel que défini à la revendication 3.

**5.** Composition selon l'une quelconque des revendications 1 à 4 dans laquelle les monomères cyanoacrylates correspondent à la formule (C) :

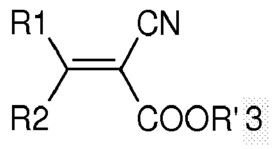

(C)

dans laquelle R'3 représente un radical alkyle en $C_1$-$C_{10}$, alcényle en $C_2$-$C_{10}$, ou alcoxy($C_1$-$C_4$)alkyle($C_1$-$C_{10}$) et R1 et R2 sont tels que définis dans la revendication 3.

**6.** Composition selon l'une quelconque des revendications précédentes, dans laquelle le monomère électrophile est un cyanoacrylate d'alkyle de formule (F) :

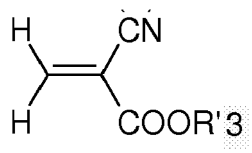

(F)

dans laquelle : $R'_3$ =$-(CH_2)_7-CH_3$,
$-CH(CH_3)-(CH_2)_5-CH_3$,
$-CH_2-CH(C_2H_5)-(CH_2)_3-CH_3$,
$-(CH_2)_5-CH(CH_3)-CH_3$,
$-(CH_2)_4-CH(C_2H_5)-CH_3$.

**7.** Composition selon l'une quelconque des revendications précédentes dans laquelle la quantité de monomères électrophile est comprise entre 0,1 et 80 % en poids du poids total de la composition.

**8.** Composition selon l'une quelconque des revendications précédentes, dans laquelle le ou les colorants directs hydrophiles comprenant un logP inférieur ou égal à 2 sont choisis parmi les colorants directs nitrés benzéniques neutres ; acides ou cationiques ; les colorants directs azoïques neutres acides ou cationiques ; les colorants directs quinoniques; les colorants directs aziniques ; les colorants directs triarylméthaniques ; les colorants directs indoaminiques et les colorants directs naturels.

**9.** Composition selon la revendication précédente dans laquelle les colorants directs hydrophiles sont choisis parmi le 4-nitro-o-phénylènediamine, 2-nitro-p-phénylènediamine, acide Picramique, HC Red 13, N,N'-bis-(2-hydroxyéthyl)-2-nitro-p-phénylènediamine, HC Red 7, HC Blue 2, HC Yellow 4, HC Red 3, 4-amino-3-nitrophénol, 1-hydroxyéthylamino-5-nitroanisole, 3-nitro-p-hydroxyéthylaminophénol, 3-méthylamino-4-nitrophénoxyéthanol, 2-nitro-5-glycéryl méthylaniline, HC Violet 1, HC Orange 2, HC Yellow 9, 4-nitrophényl aminoéthylurée, acide 2-hydroxyéthyl picramique, HC Blue 12, 3-nitro-4-N-bétahydroxyéthylaminotoluène, 2-N-béta-méthoxyéthylamino-N, Nbis-(hydroxyéthyl)amino-nitrobenzène, HC Yellow 10, HC Violet 2, 2-amino-6-chloro-4-nitrophénol, 2-amino-6-chloro-4-nitrophénol, 4-hydroxypropylamino-3-nitrophénol, HC Yellow 13, 2,6-diamino-3-((pyridine-3-yl)azo)pyridine, Disperse Black 9, HC Blue 14, HC Red 10, HC Red 11, Acid orange 7, Acid violet 43, Acid black 1, Acid red 52, Acid blue 9, Acid red 18 et Acid yellow 1.

**10.** Composition selon l'une quelconque des revendications précédentes dans laquelle le ou les colorants directs hydrophiles se trouvent dans des proportions comprises dans des quantités comprises entre 0,001 à 10 % en poids environ du poids total de la composition.

**11.** Composition selon l'une quelconque des revendications précédentes dans laquelle le milieu de coloration contient un inhibiteur de polymérisation.

**12.** Composition selon l'une quelconque des revendications précédentes comprenant de plus un agent nucléophile.

**13.** Procédé de coloration des matières kératiniques **caractérisé par le fait que** l'on applique sur les matières kératiniques en particulier les fibres kératiniques notamment les cheveux, la composition telle que définie à l'une quelconque des revendications 1 à 12 en présence d'un agent nucléophile.

**14.** Kit de coloration comprenant dans un compartiment une composition qui contient au moins un colorant direct hydrophile possédant un logP inférieur ou égal à 2 et le ou les monomères électrophiles tel que défini aux revendications 1 à 11, et dans un autre compartiment une deuxième composition qui contient un agent nucléophile, étant entendu que le solvant peut se trouver dans l'un et/ou l'autre des compartiments.

**15.** Kit de coloration comprenant dans un compartiment la composition contenant au moins un monomère électrophile tel que défini aux revendications 1 à 11, et dans un autre compartiment une deuxième composition qui contient au moins un colorant direct possédant un LogP inférieur ou égal à 2 ; étant entendu que le solvant peut se trouver dans l'un et/ou l'autre des compartiments.

**16.** Utilisation d'une composition telle que définie aux revendications 1 à 12 pour la coloration des matières kératiniques en particulier les fibres kératiniques telles que les cheveux.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2741530
- FR 2833489
- US 2748050 A
- US 3527224 A
- US 3591767 A
- US 3667472 A
- US 3995641 A
- US 4035334 A
- US 4650826 A


### Littérature non-brevet citée dans la description


- **SAYYAH.** *J. Polymer Research,* 2000, 97
- **HOPFF.** *Makromoleculare Chemie,* 1961, 95
- **DE KEYSER.** *J. Pharm. Sci,* 1991, 67
- **KLEMARCZYK.** *Polymer,* 1998, 173
- **BRETON.** *Biomaterials,* 1998, 271
- **COUVREUR.** *Pharmaceutical Research,* 1994, 1270
- **BACHRACH.** *European Polymer Journal,* 1976, 563
- **WANATABE.** *J.Polymer Science : Part A :Polymer chemistry,* 1994, 2073
- **GIPSTEIN.** *J.Org.Chem,* 1980, 1486
- **BOOR.** *J.Polymer Science,* 1971, 249
- **KANGA.** *Polymer preprints (ACS, Divison of Polymer Chemistry),* 1987, 322
- **BONNER.** *Polymer Bulletin,* 1992, 517
- **KOBAYASHI.** *Journal of Polymer Science, Part A: Polymer Chemistry,* 2005, 2754
- **ROZENBERG.** *International Journal of Plastics Technology,* 2003, 17
- **SCHMITT.** *Macromolecules,* 2001, 2115
- **LSHIZONE.** *Macromolecules,* 1999, 955
- **PR WELLS.** *Prog. Phys. Org. Chem,* 1968, vol. 6, 111
- Advanced Organic Chemistry. 151-161
- Advanced Organic Chemistry. 141
- **MEYLAN ; HOWARD.** Atom / Fragment contribution method for estimating octanol-water partition coefficient. *J. Pharm. Sci.,* 1995, vol. 84, 83-92